# EUROPEAN PATENT SPECIFICATION

(11) **EP 0 660 665 B1**
(45) Date of publication and mention of the grant of the patent: **06.05.1999**
(21) Application number: 94923320.9
(22) Date of filing: 06.07.1994
(51) Int. Cl.: A01N 1/02, A61L 2/00, A61M 1/14, B01J 19/08

(54) **APPARATUS AND METHOD FOR INACTIVATING VIRAL CONTAMINANTS IN BODY FLUIDS**
APPARATUR UND VERFAHREN ZUR INAKTIVIERUNG VON VIRALEN VERUNREINIGUNGEN IN KÖRPERFLÜSSIGKEIT
APPAREIL ET PROCEDE D'INACTIVATION DES CONTAMINANTS VIRAUX DES LIQUIDES BIOLOGIQUES

(30) Priority: 12.07.1993 US 90381
(43) Date of publication of application: 05.07.1995
(60) Divisional application: 98202100.8
(73) Proprietor: BAXTER INTERNATIONAL INC., Deerfield, Illinois 60015 (US)
(72) Inventor: WOLF, Ludwig, Jr., Barrington, IL 60010 (US); BRATTEN, William, Lake Villa, IL 60046 (US); FOLEY, John, Wheeling, IL 60090 (US)
(74) Representative: MacGregor, Gordon
(86) International application number: US9407508
(87) International publication number: WO9502325

(56) References cited:
- DE-U- 9 102 709
- US-A- 4 726 949
- US-A- 4 950 665
- US-A- 5 030 200
- Walter Reed Army Institute of Research, Research Report, WRAIR-53-55, issued November 1955, F. HEINMETS et al., "Inactivation of Viruses in Plasma by Photosensitized Oxidation", pages 1-16, see Table 1.
- Vox Sang., Volume 60, Number 4, issued May 1991, LAMBRECHT et al., "Photoinactivation of Viruses in Human Fresh Plasma by Phenothiazine Dyes in Combination with Visible Light", pages 207-213, see Abstract.
- Infusionstherapie, Volume 19, issued 1992, MOHR et al., "Virus Inactivated Single-Donor Fresh Plasma Preparations", pages 79-83, see Summary.
- Vox Sang., Volume 45, issued 1983, WALLVIK et al., "Platelet Concentrates Stored at 22 Degrees C Need Oxygen", pages 303-311, see Abstract.
- Transfusion, Volume 30, Number 6, issued 1990, NEYNDORFF et al., "Development of a Model to Demonstrate Photosensitizer-Mediated Inactivation in Blood", pages 485-490.

## Description

### BACKGROUND OF THE INVENTION

The present invention relates generally to apparatus and methods for inactivating viral contaminants that may be present in the body fluids. More specifically, the invention relates to apparatus and methods for photodynamically inactivating viral contaminants in body fluids.

In a variety of therapies, such as transfusions and transplants, body fluids, especially blood components such as red blood cells, platelets, plasma, leukocytes, and bone marrow, are collected from one or more individuals and then infused into a patient. Although such therapies provide treatments, some of which are life saving, due to the transmission of infectious diseases there may be potential risks involved with such therapies.

By way of example, it is known that blood can carry infectious agents such as hepatitis virus, human immunodeficiency virus (an etiological agent for AIDS), and herpes virus. Although screening methods exist to identify blood that may include such viruses, blood containing viruses, and other disease causing pathogens, such as bacteria, cannot be 100% eliminated from the pool of possible blood component supplies. Therefore, there is still a small risk that blood transfusions can transmit viral or other infections.

Accordingly, a goal of recent biomedical research has been to reduce the risk of transmitting an infectious agent by selectively inactivating or depleting pathogens present in such blood components. One approach has been to utilize photosensitive (photoactive) agents that when activated by light of the appropriate wavelength will destroy the ability of the pathogen to cause infection. The use of photodynamic therapy has been suggested as a way to eradicate infectious agents from collected blood and its components prior to storage and/or transfusion.

A number of different photoactive agents have been proposed as possibilities to be used to eradicate viral and other contaminants in body fluids. Such photoactive agents include: psoralens; porphyrins; phthalocyanines; and dyes such as methylene blue. See, for example, U.S. Patent Nos. 4,748,120; 4,878,891; 5,120,649; and German Patent Application No. DE 39 30 510 A1 (Mohr).

Although much effort has been focussed on commercializing such methods using photoactive agents, the inventors believe that such methods are currently not commercial. Even though a commercial system for utilizing a photoactive agent to treat blood to eradicate or remove viral and other contaminants has not been developed, it is envisioned that such a system would entail combining the blood with the photoactive agent in a container and irradiating the resultant mixture with light of the appropriate wavelength.

It is known, of course, to use blood pack units to collect blood. The blood pack units include a container typically constructed from a plastic material, usually a polyvinyl chloride material. The blood pack units are connected to tubes that allow blood to be infused into the container as well as to be accessed therefrom.

Of course, blood pack units must be sterilized. Typically, sterilization takes place by steam sterilization at a temperature of above 100°C for a predetermined period of time.

One photoactive agent that appears to be promising with respect to eradicating viruses and bacteria from blood is methylene blue. Methylene blue 3-7-bis(dimethylamino)phenothiazine-5-ium chloride (C₁₆H₁₈CIN₃S), in the presence of light has been reported to damage DNA. Accordingly, it can be used to selectively, in a controlled manner, modify the DNA and RNA of bacterial and viral contaminants thereby inactivating the pathogens. See U.S. Patent No. 4,950,665 and EP-A-0574410.

It has previously been found that methylene blue does not migrate into non-PVC material as well as into PVC material under sterilization conditions. Suitable containers providing a steam sterilizable housing for the methylene blue are disclosed in EP-A-0650345. The housing includes at least the surface that contacts the methylene blue solution being constructed from a non-PVC material.

The present invention provides a method for inactivating viruses in a body fluid, comprising the steps of:
forming a mixture including methylene blue and a body fluid in a container under sterile conditions; and
irradiating the mixture with a light field of a suitable intensity and wavelength for activating the methylene blue for a given time sufficient to inactivate viruses in the mixture, while maintaining the mixture in a no flow state within the container;
   characterised in that the container of mixture is transported on a conveyor between a pair of light sources at a speed such that at the end of the path of travel of the container , the mixture will have been irradiated for said given time.

The invention also provides apparatus according to Claim 13 for carrying out the method.

In an embodiment of the invention, the transport is configured to transport the container within the light field for at least 5 minutes.

Additional features and advantages of the present invention are described in, and will be apparent from, the detailed description of the presently preferred embodiments and from the drawings.

### BRIEF DESCRIPTION OF THE DRAWINGS

Figure 1 illustrates a perspective view of a device for irradiating a container with a light field.
Figure 2 illustrates a fragmentary cross section of the device of Figure 1.
Figure 3 illustrates a system for irradiating a container with a light field and transporting the container through the light field.

### DETAILED DESCRIPTION OF THE PRESENTLY PREFERRED EMBODIMENTS

The present invention provides apparatus and methods for inactivating pathogens that may be contained in the body fluids. As used herein, body fluid not only includes blood and its components, but also includes other fluids contained in the body such as blood components, leukocytes, red blood cells, white blood cells, plasma (even fresh frozen), etc. or fluid containing structures such as, e.g., bone marrow, semen, and internal organs.

As previously noted, although body fluids, such as blood and its components, can be used in many therapeutic applications, there is the danger of the transfer of infectious disease due to viral and bacterial contaminants that may be contained in such fluids. Recently the use of photoactive agents has provided the hope of inactivating viral and bacterial contaminants that may be contained in such fluids. However, in order to commercialize such methods, certain obstacles must be overcome.

It is known to house blood components in plastic containers. Typically, the plastic container comprises a polyvinyl chloride structure that is plasticized with di(2-ethylhexyl)phthalate (DEHP) and includes stabilizers. Blood components are stored particularly well in such containers. However, it has been found that when a solution of methylene blue, a photoinactive agent of promise, is placed in such a container at a physiological pH of around 7, that upon steam sterilization, the photoinactive agent (methylene blue) migrates into the plastic.

Methods of use of methylene blue to inactivate viral contaminants require rather precise amounts of methylene blue. See, for example, Lambrecht, et al., *Photoinactivation of Viruses in Human Fresh Plasma by Phenothiazine Dyes in Combination with Visible Light*, Vox Sang 1991; 60:207-213. Therefore, the migration of the methylene blue solution into the plastic during the sterilization process are believed to provide an unacceptable system.

It has also been found that the migration of methylene blue into the plastic is dependent on a couple of controllable parameters. Therefore, it is possible to provide apparatus and systems wherein methylene blue can be contained within a plastic structure, the structure can be steam sterilized, and the methylene blue solution will be recovered in sufficient quantity to allow the solution to be used to inactivate viral contaminants in a body fluid.

By controlling the type of plastic from which the housing that contains the methylene blue is constructed, the migration of methylene blue into the housing can be controlled.

Of most interest are the more inert plastics, such as polyolefins and polyurethanes. In a preferred embodiment, polypropylene, styrene-ethylene-butylene-styrene (SEBS), ethylenevinyl acetate, and polyesters are used.

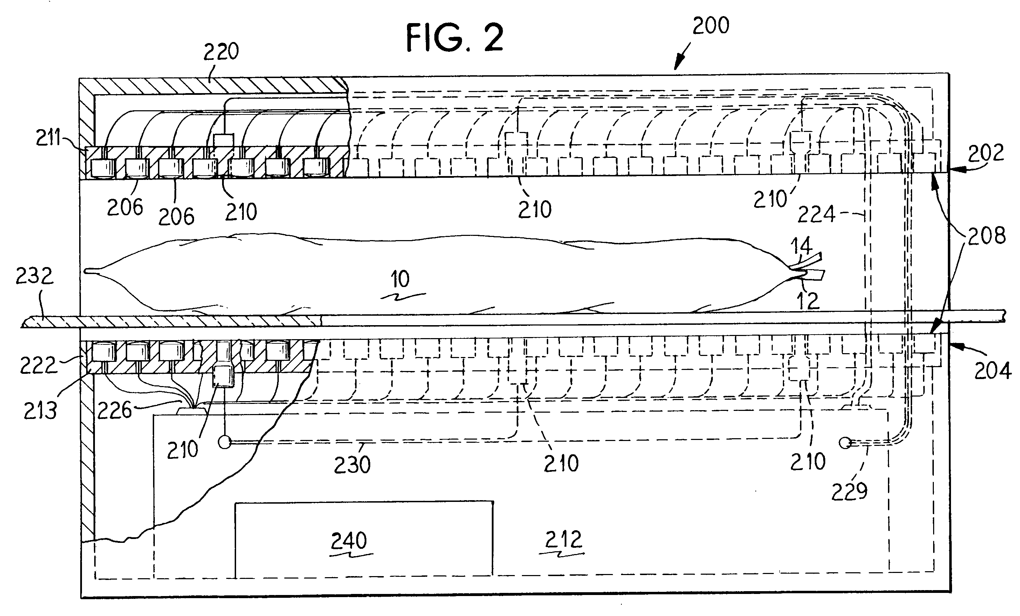

By way of example, referring first to Figure 2, a container 10 is illustrated. The container 10 includes ports 12 and 14 extending therefrom to provide access to an interior of the container 10.

In the container 10 illustrated, the ports 12 and 14 extend from the container 10 and provide means for infusing the body fluid, such as blood or blood components, into the container 10. An example of a system that can be used is the Optipak® system that is disclosed in U.S. Patent No. 4,608,178. In this system, plasma or red blood cells, for example, can be infused into the container 10 through the ports after having been separated from whole blood.

The container 10 can have a structure that is substantially similar to the container for housing blood and blood components available from the Fenwal Division of Baxter International Inc. However, the container 10 can also be constructed so that at least an interior layer that defines the interior surface of the container is constructed from a non-PVC material so as to house methylene blue alone. In that case, most preferably, at least the inner surface of the container 10 is constructed from SEBS, polypropylene, polyester, polyurethane, or ethylenevinyl acetate, or blends thereof. Of course, if desired, the entire container 10 can be constructed from a non-PVC material.

A body fluid such as a blood component and a therapeutically effective amount of methylene blue are mixed together in the container 10. In one embodiment, the methylene blue is added to body fluid already stored in the container 10. In another embodiment, the body fluid is added to a therapeutically effective amount of methylene blue already stored in the container 10. The foregoing can be accomplished by any known sterile means and/or method that has been found to function satisfactorily. In this embodiment, the amount of methylene blue solution used is about 10 ml.

It has been found that the pH of the solution can effect the migration of the solution into the plastic material during sterilization. Preferably, the methylene blue solution has been adjusted to a pH of less than 7.0 and most preferably approximately 6.3 or less.

If methylene blue solution is infused into the container 10 first, the container 10 and methylene blue solution can then be steam sterilized, e.g., at 115°C for 65 minutes. Due to the use of a methylene blue solution having a pH of less than 7.0 and the fact that the container 10 would then include at least an inner surface that is constructed from a non-PVC material, the methylene blue solution, during steam sterilization, will not substantially migrate into the plastic.

A body fluid, such as a blood component, can then be infused into the container 10 through port 14 or 16. To control fluid flow through the port, a breakable cannula or other means can be used.

In the container 10, the body fluid, preferably a blood component, will mix with the methylene blue solution. The container 10 is then irradiated by a light field of the appropriate wavelength (approximately 620-670 nm) to activate the methylene blue within the container 10. This will cause the methylene blue to inactivate any pathogens, e.g., viruses and bacteria, that are contained within the blood component, thus insuring a blood component that does not contain pathogens. The mixture is inactivated for a sufficiently long time and at a suitable wavelength to at least inactivate the viruses in the mixture.

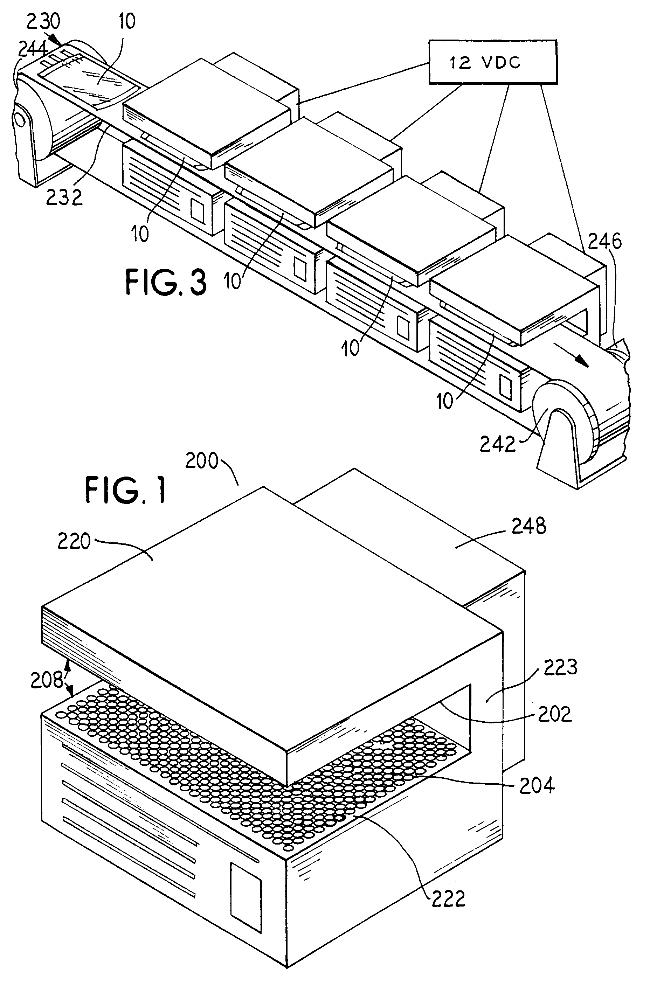

Referring now to both Figures 1 and 2, there is illustrated an apparatus 200 for generating the appropriate light field. As illustrated, the apparatus 200 preferably includes facing arrays 202 and 204 of light emitting diodes 206 that generate the light field to which the container 10 can be subjected. As will be appreciated, the apparatus 200 preferably is used for activating methylene blue in single units of fresh frozen plasma.

In an embodiment of the apparatus 200, the light emitting diodes 206 comprise light emitting diodes manufactured by Hewlett Packard under the designation HLMP-8103 and have a light output centered at a wavelength of about 670 nm. Red blood cells essentially are transparent to light radiated at this wavelength. The methylene blue, however, is not. Instead, at this wavelength the methylene blue absorbs the light and becomes activated.

In an embodiment, the light emitting diodes 206 preferably are mounted into printed circuit boards 211 and 213 about the size of 21.5 x 28 cm (8½^{"} x 11") sheets of paper. Each board then holds approximately 1280 light emitting diodes forming the relatively large arrays 202 and 204 of light emitting diodes. When powered by a 12 volt DC power supply, the pair 202 and 204 of such arrays can deliver a light field of about 300 mw per centimeter square.

As illustrated, in the apparatus 200, the two arrays 202 and 204 are mounted facing each other in spaced apart relation in such a way that a gap 208 is present within which a container containing about 250 ml of the body fluid, preferably fresh frozen plasma, and containing about 1 µM can be placed between the two arrays 202 and 204. The container can then be irradiated for about 5 minutes, which causes the methylene blue therein to inactivate any virus present.

As a result, the methylene blue and body fluid to be treated can be separately collected and sterilized, and then mixed either in the container in which the methylene blue was collected or the container in which the body fluid was collected. Then, the container can be relatively quickly irradiated by the light field generated by the apparatus 200 so that the mixture therein need not be flowed through flow paths and the like past the sources of light.

In an embodiment, the body fluid and methylene blue are contained in a PVC container. Due to the use of a PVC container, excess methylene, i.e., that methylene blue which is not activated or necessary for photoinactivation of the viruses, leaches out into the container material. Thus, the body fluid can be relatively quickly treated and excess methylene blue is removed from the body fluid.

As illustrated in Figure 2, a device such as the apparatus 200 can be equipped with light sensors 210 that can measure how much light is being delivered to the container 10 and how much light is being transmitted through the container 10 to control a total dosage of light. To this end, the apparatus can include a microprocessor based computer 212 that monitors the sensors 210 and computes cumulative dosages of light. Once a sufficient dosage is reached, the computer 212 can shut off the light emitting diodes 206.

It can be appreciated that some body fluids and/or containers are more opaque than other fluids and/or containers and that therefore the computer 212 must be programmed to accommodate the different light transmissions through the containers due to the differences in opacities of different body fluids.

As illustrated in Figures 1 and 2, in the embodiment illustrated, the apparatus 200 is constructed such that the array 202 is supported within an arm 220 that overhangs a facing support surface 222. The support surface 222 in turn supports or includes the array 204. The overhang 220 is in turn supported by perpendicular wall 223.

Interconnecting lines or cables 224 can be used to interconnect the diodes 206 of the array 202 and the computer 212 while similar lines or cables 226 can be used to interconnect the diodes 206 of the array 204 to the computer 212. In a readily programmable manner, the computer 212 can be used to turn the arrays 202 and 204 off and on as needed.

At the same time, lines or cables 228 can be used to interconnect the sensors 210 in the array 202 with the computer 212 while, similarly, lines or cables 229 can be used to interconnect the sensor 210 in the array 204 with the computer 212.

Referring now to Figure 3, it can be seen that a plurality of such apparatus 200 are placed along a path of travel defined by a conveyor 230 such that a container can be placed on a belt 232 of the conveyor 230 at one end of the conveyor 230 and transported past successive apparatus 200. It can be appreciated that the speed of the belt 232 can be adjusted such that each container 10 placed thereon would be transported past the series of light field apparatus 200 with such a timing (preferably 5 minutes or so) that at the end of the path of travel, the container 10 will have been subjected to a sufficient dosage of light to virally inactivate the body fluid contained therein.

A suitable power supply 240, e.g., a 12 volt DC power supply, can be coupled to each apparatus 200 as illustrated. Moreover, the conveyor 230 can be provided with suitable drive wheels 242 and 244 at opposite ends thereof, as well as a suitably controllable drive motor 246.

It can be appreciated that the belt 232 of the conveyor 230 should be sufficiently translucent to allow both arrays 202 and 204 to generate the appropriate light field. If the belt 232 is not sufficiently translucent, then the light field could be cut up to half and then the exposure time would have to be suitably increased.

Referring to Figure 3, it can be appreciated that all of the apparatus 200 can be connected to a single computer, perhaps even only one of the computers 212 such that the single computer monitors the total dosage of light received by each container 10 passing through the light field. The programming necessary to effect the foregoing is minimal and well within the capabilities of one of ordinary skill in the art, and therefore it is believed to not be necessary to set forth such programming herein.

## Claims

1. A method for inactivating viruses in a body fluid, comprising the steps of:
forming a mixture including methylene blue and a body fluid in a container (10) under sterile conditions; and
irradiating the mixture with a light field of a suitable intensity and wavelength for activating the methylene blue for a given time sufficient to inactivate viruses in the mixture, while maintaining the mixture in a no flow state within the container;
characterised in that the container of mixture is transported on a conveyor (230) between a pair of light sources (202,204) at a speed such that at the end of the path of travel of the container (10), the mixture will have been irradiated for said given time.

2. The method of Claim 1, wherein the body fluid is a blood component.

3. The method of Claim 2, wherein the blood component is selected from the group consisting of plasma, red blood cells, white blood cells, leukocytes, bone marrow and platelets.

4. The method of Claim 1, wherein the blood component is fresh frozen plasma.

5. The method of Claim 1, wherein the container (10) is made of a plastic.

6. The method of any preceding claim, wherein the step of forming the mixture comprises the step of adding the methylene blue to a container (10) in which the body fluid is already stored.

7. The method of any preceding claim, wherein the body fluid is stored in a container made of polyvinyl chloride material.

8. The method of any one of Claims 1 to 5, wherein the step of forming the mixture comprises the step of adding the body fluid to a container in which the methylene blue is already stored.

9. The method of any preceding claim, wherein the methylene blue is stored in a container having at least an inner surface made of a non-polyvinyl chloride material.

10. The method of Claim 1, comprising the further step of allowing excess methylene blue to leach out into the container after the irradiation step.

11. The method of Claim 1, wherein the light sources include light emitting diodes.

12. The method of Claim 1, wherein the mixture is irradiated by the light field for a cumulative period of five minutes or greater.

13. An apparatus for inactivating viruses in a body fluid with methylene blue in accordance with the method of Claim 1, comprising:
a pair of light sources (202,204) disposed so as to face each other with a gap therebetween, the light sources generating a light field of suitable wavelength and intensity to activate methylene blue; and conveyor (230) having a support surface in the gap on which a container containing a mixture of the body fluid and methylene blue can be transported between the light sources.

14. The apparatus of Claim 13, wherein the conveyor (230) comprises a belt conveyor.

15. The apparatus of Claims 13 or 14, including drive means for driving the conveyor (230) at a speed such that the container is maintained within the light field for at least five minutes.

16. The apparatus of any one of Claims 13 to 15, wherein the light sources comprise light emitting diodes.

17. The apparatus of any one of Claims 13 to 16, wherein the light sources generate a light field with a wavelength of about 670 nm.

18. The apparatus of any one of Claims 13 to 17, further comprising a sensor disposed to sense the amount of light delivered to a container on the conveyor.

19. The apparatus of Claim 18, wherein the sensor senses the amount of light transmitted through a container and the mixture contained in it.

20. The apparatus of any one of Claims 13 to 19, further comprising a processor coupled to said light sources and sensor and operable to monitor the cumulative amount of light delivered to a container.

## Patentansprüche

1. Verfahren zum Inaktivieren von Viren in einer Körperflüssigkeit, das die folgenden Schritte aufweist:
- Herstellen eines Gemischs, das Methylenblau und eine Körperflüssigkeit aufweist, in einem Behälter (10) unter sterilen Bedingungen;
- Bestrahlen des Gemischs mit einem Lichtfeld einer geeigneten Intensität und Wellenlänge, um das Methylenblau für eine gegebene Zeitdauer zu aktivieren, die ausreicht, um Viren in dem Gemisch zu inaktivieren, während gleichzeitig das Gemisch in einem strömungslosen Zustand in dem Behälter gehalten wird;
dadurch gekennzeichnet,
daß der Gemischbehälter auf einem Förderer (230) zwischen einem Paar von Lichtquellen (202, 204) mit einer solchen Geschwindigkeit transportiert wird, daß am Ende der Bewegungsbahn des Behälters (10) das Gemisch für die gegebene Zeitdauer bestrahlt worden ist.

2. Verfahren nach Anspruch 1,
wobei die Körperflüssigkeit eine Blutkomponente ist.

3. Verfahren nach Anspruch 2,
wobei die Blutkomponente aus der Gruppe ausgewählt ist, die aus Plasma, roten Blutzellen, weißen Blutzellen, Leukozyten, Knochenmark und Thrombozyten besteht.

4. Verfahren nach Anspruch 1,
wobei die Blutkomponente Fresh-frozen-Plasma (FFP) ist.

5. Verfahren nach Anspruch 1,
wobei der Behälter (10) aus einem Kunststoff besteht.

6. Verfahren nach einem der vorhergehenden Ansprüche, wobei der Schritt des Herstellens des Gemischs den Schritt aufweist, daß das Methylenblau in einen Behälter (10) zugegeben wird, in dem die Körperflüssigkeit bereits aufbewahrt wird.

7. Verfahren nach einem der vorhergehenden Ansprüche, wobei die Körperflüssigkeit in einem Behälter aufbewahrt wird, der aus Polyvinylchloridmaterial besteht.

8. Verfahren nach einem der Ansprüche 1 bis 5,
wobei der Schritt des Herstellens des Gemischs den Schritt aufweist, daß die Körperflüssigkeit in einen Behälter zugegeben wird, in dem das Methylenblau bereits aufbewahrt wird.

9. Verfahren nach einem der vorhergehenden Ansprüche, wobei das Methylenblau in einem Behälter aufbewahrt wird, der wenigstens eine innere Oberfläche hat, die aus einem Nicht-Polyvinylchloridmaterial besteht.

10. Verfahren nach Anspruch 1,
das den weiteren Schritt aufweist, daß man überschüssiges Methylenblau nach dem Bestrahlungsschritt in das Behältermaterial hinein austreten läßt.

11. Verfahren nach Anspruch 1,
wobei die Lichtquellen lichtemittierende Dioden aufweisen.

12. Verfahren nach Anspruch 1,
wobei das Gemisch von dem Lichtfeld für einen kumulativen Zeitraum von fünf Minuten oder mehr bestrahlt wird.

13. Vorrichtung zum Inaktivieren von Viren in einer Körperflüssigkeit mit Methylenblau nach dem Verfahren gemäß Anspruch 1, wobei die Vorrichtung folgendes aufweist:
- ein Paar von Lichtquellen (202, 204), die so angeordnet sind, daß sie mit einem Zwischenraum zwischeneinander einander gegenüberstehen, wobei die Lichtquellen ein Lichtfeld von geeigneter Wellenlänge und Intensität erzeugen, um Methylenblau zu aktivieren; und
- einen Förderer (230) mit einer Tragfläche in dem Zwischenraum, auf der ein Behälter, der ein Gemisch aus der Körperflüssigkeit und Methylenblau enthält, zwischen den Lichtquellen transportiert werden kann.

14. Vorrichtung nach Anspruch 13,
wobei der Förderer (230) einen Bandförderer aufweist.

15. Vorrichtung nach Anspruch 13 oder 14,
die eine Antriebseinrichtung aufweist, um den Förderer (230) mit einer solchen Geschwindigkeit anzutreiben, daß der Behälter für eine Dauer von wenigstens fünf Minuten in dem Lichtfeld gehalten wird.

16. Vorrichtung nach einem der Ansprüche 13 bis 15, wobei die Lichtquellen lichtemittierende Dioden aufweisen.

17. Vorrichtung nach einem der Ansprüche 13 bis 16,
wobei die Lichtquellen ein Lichtfeld mit einer Wellenlänge von ca. 670 nm erzeugen.

18. Vorrichtung nach einem der Ansprüche 13 bis 17,
die ferner einen Sensor aufweist, der so angeordnet ist, daß er die an einen Behälter auf dem Förderer abgegebene Lichtmenge mißt.

19. Vorrichtung nach Anspruch 18,
wobei der Sensor die Lichtmenge mißt, die durch einen Behälter und das darin enthaltene Gemisch durchgelassen wird.

20. Vorrichtung nach einem der Ansprüche 13 bis 19,
die ferner einen Prozessor aufweist, der mit den Lichtquellen und dem Sensor verbunden und wirksam ist, um die an einen Behälter abgegebene kumulative Lichtmenge zu überwachen.

## Revendications

1. Procédé d'inactivation des virus dans un fluide biologique, comprenant les étapes de :
préparation d'un mélange comprenant du bleu de méthylène et un fluide biologique dans un récipient (10) dans des conditions stériles ; et
irradiation du mélange avec un champ de lumière d'une intensité et d'une longueur d'onde appropriées pour activer le bleu de méthylène,pendant un temps donné suffisant pour inactiver les virus présents dans le mélange, tout en maintenant le mélange dans un état sans écoulement à l'intérieur du récipient ;
caractérisé en ce que le récipient de mélange est transporté sur un convoyeur (230) entre deux sources de lumière (202, 204) à une vitesse telle que, à l'extrémité du chemin de déplacement du récipient (10), le mélange a été irradié pendant le dit temps donné.

2. Procédé selon la revendication 1, dans lequel le fluide biologique est un composant du sang.

3. Procédé selon la revendication 2, dans lequel le composant du sang est choisi dans le groupe comprenant : plasma, globules rouges, globules blancs, leucocytes, moelle osseuse et plaquettes.

4. Procédé selon la revendication 1, dans lequel le composant du sang est du plasma frais gelé.

5. Procédé selon la revendication 1, dans lequel le récipient (10) est en matière plastique.

6. Procédé selon une quelconque des revendications précédentes, dans lequel l'étape de préparation du mélange comprend l'étape d'addition du bleu de méthylène à un récipient (10) dans lequel le fluide biologique est déjà stocké.

7. Procédé selon une quelconque des revendications précédentes, dans lequel le fluide biologique est stocké dans un récipient en matière de type chlorure de polyvinyle.

8. Procédé selon une quelconque des revendications 1 à 5, dans lequel l'étape de préparation du mélange comprend l'étape d'addition du fluide biologique à un récipient dans lequel le bleu de méthylène est déjà stocké.

9. Procédé selon une quelconque des revendications précédentes, dans lequel le bleu de méthylène est stocké dans un récipient ayant au moins une surface intérieure en matière non de type chlorure de polyvinyle.

10. Procédé selon la revendication 1, comprenant en outre l'étape de permettre au bleu de méthylène en excès de migrer dans le récipient après l'étape d'irradiation.

11. Procédé selon la revendication 1, dans lequel les sources de lumière comprennent des diodes émettrices de lumière.

12. Procédé selon la revendication 1, dans lequel le mélange est irradié par le champ de lumière pendant une période cumulée de cinq minutes ou plus.

13. Appareil pour l'inactivation des virus dans un fluide biologique au moyen de bleu de méthylène selon le procédé de la revendication 1, comprenant :
deux sources de lumière (202, 204) disposées en regard l'une de l'autre avec un intervalle entre elles, les sources de lumière engendrant un champ de lumière de longueur d'onde et d'intensité appropriées pour activer le bleu de méthylène ; et
un convoyeur (230) ayant une surface de support dans l'intervalle, sur lequel un récipient contenant un mélange du fluide biologique et du bleu de méthylène peut être transporté entre les sources de lumière.

14. Appareil selon la revendication 13, dans lequel le convoyeur (230) comprend un convoyeur à courroie.

15. Appareil selon les revendications 13 ou 14, incluant des moyens d'entraînement pour entraîner le convoyeur (230) à une vitesse telle que le récipient est maintenu dans le champ de lumière pendant au moins cinq minutes.

16. Appareil selon une quelconque des revendications 13 à 15 , dans lequel les sources de lumière comprennent des diodes émettrices de lumière.

17. Appareil selon une quelconque des revendications 13 à 16, dans lequel les sources de lumière engendrent un champ de lumière ayant une longueur d'onde de 670 nm environ.

18. Appareil selon une quelconque des revendications 13 à 17, comprenant en outre un capteur disposé pour détecter la quantité de lumière fournie à un récipient sur le convoyeur.

19. Appareil selon la revendication 18 , dans lequel le capteur détecte la quantité de lumière transmise à travers un récipient et le mélange contenu dans celui-ci.

20. Appareil selon une quelconque des revendications 13 à 19, comprenant en outre un processeur couplé aux dites sources de lumière et aux capteurs, et pouvant fonctionner pour contrôler la quantité cumulée de lumière fournie à un récipient.
